# EUROPÄISCHE PATENTANMELDUNG

(11) **EP 4 467 659 A1**
(43) Veröffentlichungstag der Anmeldung: **27.11.2024**
(21) Anmeldenummer: 23175864.0
(22) Anmeldetag: 26.05.2023
(51) Int. Cl.: C12Q 1/6806

(54) **REFERENZMATERIAL AUFWEISEND NUKLEINSÄUREN MIT EINEM FRAGMENTIERUNGS- UND NUKLEOSOMPROFIL UND DIAGNOSTIK**

(71) Anmelder: SensID GmbH, 18057 Rostock (DE)
(72) Erfinder: Nowack, Björn, 18119 Seebad-Warnemünde (DE); Lüking, Angelika, 14974 Ludwigsfelde (DE); Pielka, Anna-Maria, 18057 Rostock (DE)
(74) Vertreter: Simandi, Claus

(57) **Zusammenfassung**

Die vorliegende Erfindung betrifft ein neues Referenzmaterial aufweisend Nukleinsäuren mit einem bestimmten Fragmentierungsmuster, insbesondere zur Verwendung in Sequenzierverfahren, solche wie Polymerase-Chain-Reaction (PCR)oder Next Generation Sequencing (NGS), Verfahren zu dessen Herstellung und Verwendung sowie eine zugehörige Diagnostik. Dieses Referenzmaterial erlaubt die Validierung von solchen Verfahren einschließlich der Verifizierung von Messergebnissen, insbesondere in Abhängigkeit von Geräteparametern und Probenvorbereitung. Weiterhin umfasst die Erfindung ein zugehöriges Diagnoseverfahren zu einer Liquid Biopsy bzw. zellfreier DNA.

## Beschreibung

Die vorliegende Erfindung betrifft ein neues Referenzmaterial aufweisend Nukleinsäuren mit einem bestimmten Fragmentierungsmuster, insbesondere zur Verwendung in Sequenzierverfahren, solche wie Polymerase-Chain-Reaction (PCR)oder Next Generation Sequencing (NGS), Verfahren zu dessen Herstellung und Verwendung sowie eine zugehörige Diagnostik. Dieses Referenzmaterial erlaubt die Validierung von solchen Verfahren einschließlich der Verifizierung von Messergebnissen, insbesondere in Abhängigkeit von Geräteparametern und Probenvorbereitung. Weiterhin umfasst die Erfindung ein zugehöriges Diagnoseverfahren zu einer Liquid Biopsy bzw. zellfreier DNA.

Gegenwärtige Verfahren zum Nachweis von Krankheiten wie klinisch-chemische Untersuchungen in Körperflüssigkeiten und Gewebeproben oder bildgebende Verfahren führten trotz aller Fortschritte in den letzten Jahren zu einem meist zu späten Nachweis von nachteiligen Veränderungen. Um das Auftreten von z.B. Metastasen bei Tumorerkrankungen zu verhindern, ist ein so früh wie möglicher Nachweis von malignen Veränderungen erforderlich. Ferner bedarf es Verfahren, um kranke Zellen von gesunden Zellen richtig unterscheiden zu können. Sowohl falsch-positive als auch falsch-negative Befunde haben fatale Konsequenzen für die Betroffenen. Ebenso sind Verfahren erforderlich, die eine richtige Prognose gestatten und eine entsprechend auf den Patienten individuell abgestimmte Therapie erlauben.

Die Isolierung, Charakterisierung und Analyse von Nukleinsäuren (Ribonukleinsäuren, RNA und Desoxyribonukleinsäuren, DNA) aus Zellmaterialien erfolgt im Stand der Technik zumeist in den nachfolgenden Schritten: Probenaufbereitung, Extraktion, Konzentration, Isolierung, Reinigung, ggfs. reverse Transkription, Amplifikation und Detektion.

Aufgrund der leistungsfähigen Amplifikation von DNA mittels Polymerase-Chain-Reaction (PCR), wie verwendet bei der "qPCR" (real-time quantitative Polymerase Chain Reaction), "digitalen PCR" (dPCR), oder diverse Varianten von "next generation sequencing" (NGS, bzw. Parallelsequenzierung wie "Massive Parallel Sequencing"), hat sich die so genannte minimal bis nicht-invasive Liquid Biopsy in der Diagnostik etabliert, wobei z.B. zirkulierende freie DNA in einer Probe (Blut, Plasma) untersucht wird. Solche zirkulierende freie DNA (kurz: cfDNA) sind kurze doppel- oder einzelsträngige DNA-Moleküle (50-200 bp) aus der Fragmentierung genomischer DNA von apoptotischen und nekrotischen Zellen, und werden als Biomarker zur Diagnose von Krankheiten eingesetzt.

Bei Krebspatienten u.a. Krankheiten liegt die durchschnittliche cfDNA Menge höher als in gesunden Probanden (so genannte: ctDNA bei Tumorerkrankungen), insbesondere ist das cfDNA Plasma Level bei Krebspatienten im fortgeschrittenen Stadium höher als im milden/frühen Stadium. ctDNA macht z.B. 1% der cfDNA aus (0,001-90% der normalen cfDNA, je nach Stadium und Lokalisation des Tumors).

Es ist weiterhin bekannt, dass cfDNA im Blut oder Plasma zirkuliert, die von verschiedenen Zelltypen stammt, z.B. aus verschiedenen gesunden Organen/Geweben oder krankhaft veränderten Organen/Geweben, z.B. Tumore an unterschiedlichen anatomischen Stellen. Interessanterweise wurde auch festgestellt, dass zirkulierende fetale cfDNA (Ursprung ist hauptsächlich die Plazenta) und Tumor-ctDNA kürzer sind als die "Hintergrund"-cfDNA, die hauptsächlich hämatopoetischen Ursprungs ist. Die cfDNA trägt auch Signaturen ihrer Assoziation mit Nukleosomen und Chromatosomen, wie z. B. die am häufigsten vertretene Plasma-DNA-Länge von 166-bp und Fragmentierungsendpunkte, die Beziehungen zur Nukleosomenanordnung zeigen.

Als Nukleosom bezeichnet man die Einheit von DNA und einem Histonoktamer. Das Oktamer besteht aus je zwei Exemplaren der Proteine H2A, H2B, H3 und H4. Um so einen Proteinkomplex sind 146 oder 147 Basenpaare der DNA als linksgängige Superhelix gewunden. Als Chromatosom bezeichnet man das Ergebnis der Bindung von Histon H1 an ein Nukleosom. Das Chromatosom enthält 166 Basenpaare DNA. Im Folgenden jedoch zusammen als Nukleosom(en) bezeichnet. In allen Eukaryonten liegt die DNA in einem kondensierten Zustand vor, der als Chromatin bezeichnet wird.

Während cfDNA, die das gesamte Genom überspannt (sowohl vom nukleären als auch vom mitochondrialen Genom), im Plasma vorhanden ist, gibt es Mikroheterogenitäten in der cfDNA-Abdeckung zwischen verschiedenen genomischen Regionen. Beispielsweise unterscheiden sich die Abundanz der cfDNA und die Fragment-Endhäufigkeiten zwischen offenen und geschlossenen Chromatindomänen, Regionen innerhalb und zwischen der Nukleosomen und bestimmten genetischen Elementen wie Transkriptionsfaktorbindungsstellen. Es gibt Hinweise darauf, dass cfDNA eine Prädisposition hat, an bestimmten genomischen Regionen oder Elementen geschnitten zu werden und dadurch eine höhere Repräsentation bestimmter Endsequenz-Motive aufweist. Eine solche Selektivität der Schnittstellen kann mit den Sequenzmotivpräferenzen der am Prozess beteiligten Nukleasen in Verbindung gebracht werden. Diese Zusammenhänge sind Gegenstand der so genannten "Fragmentomics".

Die Chromatinstruktur beeinflusst die Länge und Häufigkeit der cfDNA. Offene (Euchromatin) und geschlossene Chromatinregionen (Heterochromatin) unterscheiden sich durch Häufigkeit des Auftretens von Nukleosomen und des Genexpressionsniveaus (z. B. durch das Vorhandensein von Transkriptionsfaktor(TF)-Bindungsstellen), was wiederum die Zugänglichkeit für Nukleasen erhöhen oder verringern kann. Diese Variablen manifestieren sich in den fragmentomischen Merkmalen einer cfDNA. Die Mehrheit der cfDNA ist mononukleosomal lang, und die Nukleosomen beeinflussen die Länge der cfDNA (Nukleosom-Footprint).

Das Verständnis der Fragmentierungsmuster von cfDNA in Abhängigkeit vom Zelltyp kann die Identifizierung von Pathologien/Krankheiten unterstützen, die mit diesen Geweben oder Organen verbunden sind. Weiterhin wurde kürzlich gezeigt, dass sich die Fragmentierungsmuster je nach Herkunftsgewebe (d. h. fötal oder mütterlich, Krebs oder Nicht-Krebs) oder nukleosomaler Verpackung der DNA unterscheiden können. Daher umfasst das Fragmentierungsmuster nicht nur die Fragmentgrößen, sondern auch die Fragment-Endpunkte sowie die Nukleosom-Footprints. Weiterhin ist die Genexpression beeinflusst, d.h. die Zahl der Kopien bzw. RNA-Transkripte.

Zu den großen Herausforderungen der Fragmentomics gehören jedoch präanalytische Aspekte wie die Blutentnahme und der zeitliche Ablauf in Bezug auf den Transport des Blutes zum Labor und die Verarbeitung des Blutes, insbesondere Probenvorbereitung. Darüber hinaus können cfDNA-Extraktions- und Bibliotheksvorbereitungsprotokolle DNA-Fragmente bestimmter Größen bevorzugt abreichern oder anreichern, so dass diese Schritte für aussagekräftige DNA-Fragmentierungsanalysen sorgfältig geplant werden müssen. Die biologischen Aspekte sind beispielsweise die an der Fragmentierung beteiligten verschiedenen Nukleasen (z.B. DNA-Fragmentierungsfaktor beta, DNASE1L3 und DNASE1), die teilweise überhängende Enden mit einem definierten Sequenz- oder Basenhintergrund, sowie unterschiedliche Größen hinterlassen.

Aus diesen vorstehenden Gründen ist die cfDNA-Abdeckung bzw. Sequenztiefe (sogenannte "Coverage") nicht einheitlich, sondern spezifisch, je nach Zelltyp und Nukleosom-Footprint, sodass spezifische Fragmentierungsmuster erhalten werden.

Die "Coverage" bei DNA, insbesondere cfDNA bezieht sich auf die Anzahl der Sequenzierungs-Lesevorgänge (Reads), die für die Analyse der cfDNA verwendet werden. Die Coverage wird üblicherweise als durchschnittliche Anzahl der Reads definiert, die über ein bestimmtes Genom- oder Zielgebiet hinweg generiert werden. Eine hohe Coverage bedeutet, dass eine ausreichende Anzahl von Reads vorhanden ist, um genaue Informationen über die zugrunde liegende DNA oder cfDNA-Sequenz zu erhalten. Eine niedrige Coverage kann hingegen zu ungenauen oder fehlenden Informationen führen.

Eine ausreichende Coverage ist wichtig, um seltene genetische Varianten, Mutationen oder strukturelle Veränderungen in der DNA oder cfDNA zu identifizieren. Sie ermöglicht auch eine präzise Quantifizierung bestimmter DNA-Sequenzen oder Analyse der zellfreien DNA-Fragmente im Allgemeinen.

Die genaue Coverage-Anforderung kann je nach Art der Analyse, der Fragestellung und der verwendeten Sequenzierungstechnologie variieren.

Es besteht daher ein hohes Bedürfnis an einem geeigneten Standard bzw. Referenzmaterial, so dass eine Validierung oder Kalibrierung einer cfDNA-Bestimmung in Abhängigkeit der Coverage-Anforderung eines Sequenzierungsverfahrens, oder diesbezügliche Amplikon- bzw. Nicht-Amplikonverfahren, solche wie Polymerase-Chain-Reaction (PCR)oder Next Generation Sequencing (NGS)durchführbar sind. Weiterhin sind Geräteparameter von Bedeutung.

Im Stand der Technik sind übliche Laborstandards beschrieben.

Nachteilig ist jedoch, dass diese Labor-internen Standards zwecks Validierung oder Kalibrierung eines Gerätes oder eines Verfahrens selbst hergestellt und verdünnt werden müssen und hierfür kein automatisierter Prozess zur Verfügung steht, sodass eine Standardisierung zumeist fehlerbehaftet ist. Folglich kann die mittels Sequenzierung, oder mittels Zyklen oder nicht Amplikon-basierter Verfahren ermittelte Kopienzahl mangels hinreichender Standardisierung relativ und absolut falsch sein. Weiterhin wird zumeist keine cfDNA eingesetzt, da diese aus Blutproben gewonnen werden müssten, und folglich ungenügend analysierbare Sequenzen vorliegen können. Die (Blut-)Spender können zudem krank oder gesund sein und auf diese Weise den Standard beeinflussen und verfälschen.

Daher ist es erforderlich artifizielle cfDNA als Referenzmaterial herzustellen, welches bisher im Stand der Technik nicht beschrieben ist.

Daher gilt es, die Standardisierung mit Hilfe eines artifiziellen Referenzmaterials weiter zu verbessern, welches der natürlichen cfDNA entspricht, und ein natürliches Fragmentierungsmuster (supra) aufweist und der Coverage-Anforderung genügt.

Die Aufgabe der vorliegenden Erfindung ist daher die Bereitstellung eines verbesserten Verfahrens zur Validierung eines Sequenzierverfahrens für die Bestimmung von natürlicher cfDNA.

Überraschenderweise konnten die Erfinder feststellen, dass zu einem solchen Standard die Verwendung von cfDNA mit einem bestimmten Fragmentierungsmuster die Validierung erheblich verbessert. Hierzu ist es erforderlich, die vorgenannte Coverage zu berücksichtigen, sodass in Abhängigkeit vom Zelltyp bzw. Organ oder Gewebe ein verbessertes Validierungsergebnis erreicht werden kann. Zudem müssen genügend analysierbare Sequenzen vorliegen.

Die Aufgabe wird daher gelöst durch ein Verfahren zur Validierung eines Sequenzierverfahrens für die Bestimmung von cfDNA, wobei
i.) ein Referenzmaterial bereitgestellt wird, welches mit den folgenden Schritten hergestellt wird:
   a.) Bereitstellen einer Zellkultur aufweisend Zellen,
   b.) Behandlung der Zellkultur aus a.) mit einem Hemmstoff zur Zellteilung,
   c.) Isolierung der Nukleinsäuren aufweisend Nukleosomen,
   d.) Behandlung der Nukleinsäuren aufweisend Nukleosomen mit Nukleasen und / oder Ultraschall,
   e.) Isolierung von Nukleinsäuren mit einer Länge von 20-750 bp, und
ii.) die Nukleinsäuren aus e.) mittels des Sequenzierverfahrens bestimmt werden.

Das vorstehende Verfahren wird nachstehend "erfindungsgemäßes Validierungsverfahren" genannt.

Besonders vorteilhaft kann mittels Schritt b.) die "Coverage" bzw. das gewünschte Fragmentierungsmuster erhalten bzw. beeinflusst werden. Der Hemmstoff zur Zellteilung friert den Zustand bzw. eine gewählte Coverage ein.

Bei dem erfindungsgemäßen Verfahren ist vorteilhaft, dass das Referenzmaterial in möglichst gleicher Qualität über alle Herstellzyklen hergestellt werden kann. Dies ist im Besonderen schwierig, da sich jede Zelle in jener Zellkultur, in einem unterschiedlichen Zellzyklusstadium befindet. Infolgedessen hat eine Zelle nicht den gleichen DNA-Gehalt im jeweiligen Moment ihrer Zellernte, wie eine andere Zelle. Ebenso ist die Verteilung von Hetero- und Euchromatin zwischen den Zellen unterschiedlich mit Wirkung auf das Transkriptom und dem Methylierungsmuster. Um diesem Umstand entgegenzuwirken, können die Zellen erfindungsgemäß mit einem Hemmstoff zur Zellteilung behandelt werden, welcher zum Ziel hat, alle Zellen in den gleichen Zellzyklus zum Zeitpunkt der Ernte zu überführen.

Um vorteilhaft die gesamte DNA auf Nukleosomen gepackt in Zellkultur zu erhalten, werden die Zellen vorzugsweise mit einem Mitose-Hemmstoff, vorzugsweise Colcemide (auch Colchicin genannt) behandelt, sodass die Mitose in der Metaphase gestoppt wird. Dies ist vorteilhaft, sodass eine möglichst hohe Coverage erhalten wird. Weiterhin wird vorteilhaft eine gleichmäßige Herstellung des Referenzmaterials über jeden Batch erreicht, da die Zellen immer im gleichen Zellzyklus geerntet werden können. Wenn Colchicin zu kultivierten eukaryontischen Zellen hinzugefügt wird, treten die Zellen in die Mitose ein und arretieren in der Metaphase mit kondensierten Chromosomen.

Daher können im Sinne dieser Erfindung solche Hemmstoffe zur Zellteilung, insbesondere Mitose-Hemmstoff oder Checkpoint Inhibitoren eingesetzt werden, insbesondere ausgewählt aus der Gruppe Colcemide (CAS-Nummer: 477-30-5), Benomyl (CAS Nummer: 17804-35-2), Nocodazole (CAS Nummer: 31430-18-9), Paclitaxel (CAS-Nummer: 33069-62-4), Vincristine (CAS-Nummer: 57-22-7), Vinblastine (CAS-Nummer: 865-21-4), 8-Chloroadenosine (CAS Nummer: 34408-14-5), WYE 687 dihydrochloride (CAS Nummer: 1702364-87-1), AZD 5438 (CAS Nummer: 602306-29-6), Pladienolide B (CAS Nummer: 445493-23-2), Temsirolimus (CAS Nummer: 162635-04-3), Tosyl-L-Arginine Methyl Ester (TAME) (CAS-Nummer: 1784-03-8), YC 1 (CAS Nummer: 170632-47-0), Indisulam (CAS Nummer: 165668-41-7).

Weiterhin werden erfindungsgemäße Nukleasen gemäß Schritt d.) verwendet, ausgewählt aus der Gruppe MNase (EC Nummer: 3.1.31.1), DNase1L3 (EC-Nummer: 3.1.21.1), DNase I (EC-Nummer: 3.1.21.1) .

Im Anschluss an den enzymatischen Verdau kann eine Aufreinigung der DNA erfolgen und optional eine end repair Reaktion durch Klenow/T4 DNA Polymerase (EC-Nummer: 2.7.7.7). Alternativ kann Ultraschall gemäß Schritt d.) eingesetzt werden, sodass Nukleinsäuren mit einer Länge von 20-750 bp erhalten werden können.

Bevorzugt ist jedoch der Einsatz von Nukleasen gemäß Schritt d.).

Im Sinne dieser Erfindung bedeutet "Referenzmaterial" eine in den Schritten a.)-e.) hergestellte Nukleinsäure mit einer Länge von 20-750 bp, insbesondere 50-500 bp, insbesondere 20-200 bp. Diese hergestellten Nukleinsäuren sind "artifizielle cfDNA" im Sinne dieser Erfindung.

In einer weiteren Ausführungsform der Erfindung kann die Zellkultur aufweisend Zellen gemäß Schritt a.) ein oder mehrere bestimmte Zelltypen aufweisen, insbesondere solche die zur mitotischen oder meiotischen Zellteilung fähig sind, ausgewählt aus der Gruppe der humanen Zellen, Säugetierzellen, eukaryotischen Zellen, Körperzellen, Stammzellen, B-Zellen, T-Zellen, natürliche Killerzellen, kranke oder gesunde Zellen, Tumorzellen, Zellen aus einem Gewebe oder Organ. Ebenfalls umfasst sind monoklonale Zellkulturen immortaler (unsterblicher) eukaryotischer Zellen, insbesondere solche die aus polyklonalen Kulturen durch Vereinzelung hervorgegangen sind. Immortalisierte Zellen können durch Infektion mit Viren, z.B. Infektion von Lymphozyten mit Eppstein Barr Virus, entstehen oder durch die Kultivierung von Tumorzellen, welche aus einem erkrankten Säugetier entfernt wurden. Dabei erhält man zunächst eine Mischkultur vieler Einzelzellen (sog. polyklonale Kultur). Durch Techniken zur Vereinzelung können aus einzelnen Zellen neue Zellkulturen entstehen (sog. monoklonale Kulturen). Durch die Vereinzelung werden Unterschiede, welche auf DNA-Ebene zwischen Zellen einer polyklonalen Kultur auftreten können, aufgehoben. Dadurch wird das Material besonders sauber und kann insbesondere für die Validierung hochsensitiver Verfahren eingesetzt werden. Besonders bevorzugt sind daher solche monoklonale Zellkulturen bzw. immortalisierte Zellen, aufgrund der Identität bzw. Homogenität der Zellen. Folglich weist das erfindungsgemäß erhaltene Referenzmaterial wenig Verunreinigungen auf.

Das erfindungsgemäße Verfahren erlaubt vorteilhaft die Bereitstellung eines Referenzmaterials, welches das typische und spezifische Fragmentierungsmuster wiedergibt.

Im Sinne dieser Erfindung bedeutet "Sequenzierverfahren" jedes geeignete Sequenzierverfahren zur Sequenzierung von Nukleinsäuren, DNA, insbesondere cfDNA, insbesondere nach der Sangermethode oder sogenannte 2. Generation oder Amplikon- oder Nicht-Amplikonverfahren. Amplikon- oder Nicht-Amplikonverfahren sind solche wie Pyrosequenzierung, Sequenzierung durch Hybridisierung, Ionen-Halbleiter-DNA-Sequenzierungssystem, Nanoporen-Sequenzierung, Next Generation Sequencing (NGS), sequencing-by-synthesis (SBS), Polymerase-Chain-Reaction (PCR), qPCR (real-time quantitative Polymerase Chain Reaction), digitale PCR (dPCR).

Im Sinne dieser Erfindung bedeutet Amplikonverfahren solche die für die Sequenzierung ein Amplikon benötigen. Hier sind einige gängige Sequenzierverfahren, die für Amplikone verwendet werden:
i.) Sanger-Sequenzierung: Dieses klassische Sequenzierverfahren basiert auf der Verwendung von Terminierungs-Nukleotiden, um die DNA-Sequenz zu bestimmen. Die Sanger-Sequenzierung wird oft für die Analyse von kurzen Amplikonen verwendet, typischerweise bis zu einigen hundert Basenpaaren.
ii.) Next-Generation Sequencing (NGS): NGS-Technologien wie die Illumina-Sequenzierung oder die Ion Torrent-Sequenzierung ermöglichen die Sequenzierung von vielen Amplikonen gleichzeitig. Mit NGS können Hunderttausende bis Millionen von Reads generiert werden, was zu einer hohen Durchsatzrate führt. NGS eignet sich für die Sequenzierung von Amplikonen verschiedener Längen, von einigen hundert bis zu mehreren tausend Basenpaaren.
iii.) PacBio-Sequenzierung: Die Einzelmolekül-Sequenzierungstechnologie von Pacific Biosciences (PacBio) ermöglicht die Sequenzierung langer Amplikone. Sie bietet eine sehr lange Leselänge und eignet sich daher gut für die Sequenzierung von Amplikonen, die mehrere tausend bis zehntausend Basenpaare umfassen.
iv.) Oxford Nanopore-Sequenzierung: Die Oxford Nanopore-Sequenzierung ermöglicht ebenfalls die Sequenzierung langer Amplikone. Die Technologie basiert auf der Durchführung der Sequenzierung in Echtzeit, indem die DNA-Moleküle durch eine Nanopore geführt werden. Sie bietet eine lange Leselänge und ist portabel einsetzbar.

Im Sinne dieser Erfindung bedeutet Nicht-Amplikonverfahren solche Sequenzierverfahren, die keine Amplikone benötigen.

Im Sinne dieser Erfindung bedeutet Validierung, dass anhand einer Validierungsprobe aufweisend die nach dem erfindungsgemäßen Verfahren a.) bis e.) hergestellten Nukleinsäuren (bzw. Referenzmaterial), diese mittels Sequenzierung bestimmt werden können. Dieser positive Nachweis kann einerseits von Geräteparametern abhängen, andererseits von der Durchführung der Sequenzierverfahren. Die Validierungsprobe kann mittels eines Kits bereitgestellt werden.

Sofern die Validierungsprobe bei der Durchführung des Sequenzierverfahrens nicht wiedergefunden wird, ist die Sensitivität nicht gegeben bzw. die Detektionsgrenze zu hoch, so dass ein Nachweis nicht erfolgen kann.

Eine weitere besondere Ausführungsform der Erfindung betrifft ein Verfahren zur Diagnose oder Prognose einer Erkrankung, wobei eine Bestimmung von cfDNA erfolgt, insbesondere zur Beurteilung des Schweregrades und zur therapiebegleitenden Verlaufsbeurteilung, zur Untersuchung minimaler Resterkrankung (engl. minimal residual disease) und Therapieentscheidung, wobei eine Kalibrierung mit Hilfe des erfindungsgemäßen Validierungsverfahrens erfolgt ist.

Das erfindungsgemäße Verfahren erlaubt besonders vorteilhaft, die Nachweisgrenze der cfDNA zu validieren. Eine besonders vorteilhafte Anwendung betrifft daher die Validierung von Geräten zur Durchführung insbesondere einer Polymerase-Chain-Reaction (PCR), next generation sequencing (NGS), wobei das erfindungsgemäße Verfahren durchgeführt wird.

Besonders vorteilhaft kann auf diese Weise eine exakt vorgegebene Menge an DNA-Material bzw. Konzentration des erfindungsgemäßen Referenzmaterial in eine Probe oder Vergleichsprobe eingebracht werden, wobei diese Probe eine Probe weitgehend simuliert und für die Durchführung einer Diagnostik hochgradig geeignet ist.

Der Begriff der Diagnose umfasst die medizinische Diagnostik und diesbezügliche Untersuchungen, insbesondere die in-vitro Diagnostik und Labordiagnostik. Bevorzugt ist die Aussage über eine Krankheit oder einen Zustand eines Patienten, welche aus der Bestimmung von cfDNA erfolgen kann.

Im Rahmen dieser Erfindung wird unter "Patient" ein beliebiger Proband verstanden.

Nachfolgend wird die Erfindung durch Beispiele und Figuren erläutert. Die Erfindung ist jedoch nicht auf die Beispiele und Figuren beschränkt, sondern grundsätzlich universell anwendbar.

Beispiel 1: Validierung eines diagnostischen Verfahrens zur Erkennung einer minimalen Resterkrankung (engl. = Minimal Residual Disease (MRD)) oder monitoring/follow up Untersuchungen bei Krebserkrankungen.

In einer Studie wurde fragmentierte DNA in einem NGS-Verfahren eingesetzt, um diese zu validieren. Zum Vergleich wurden verschiedene andere Materialien nicht natürlichen Ursprungs eingesetzt, mit denen ebenfalls versucht wurde das System zu validieren. Als Normalkontrolle wurde isolierte zellfreie DNA eines gesunden Spenders verwendet. Es zeigte sich, dass die DNA, hergestellt wie im beschriebenen Verfahren, zur Validierung des Systems genutzt werden konnte, während andere Materialien aufgrund ihrer Verschiedenheit zum Patientenmaterial ungeeignet waren.

Insbesondere wurde die Fragmentgrößenverteilung und Konversionseffizienz untersucht. Unter der Konversionseffizienz wird die erfolgreiche Prozessierung der DNA-Fragmente verstanden, um sie für die Sequenzierung vorzubereiten.

Damit die Validierung eines Systems, insbesondere zur Detektion einer MRD, erfolgreich durchgeführt werden kann, muss das verwendete Material zur Validierung dem Patientenmaterial sehr ähnlich sein und besonders sauber (eine niedrige Fehlerrate aufweisen).

Es zeigte sich, dass sowohl die Konversionseffizienz als auch die Größenverteilung des Materials, wenn es erfindungsgemäß hergestellt wird, dem Patientenmaterial so ähnlich ist, dass eine Validierung des Systems erfolgreich durchgeführt werden kann.

Alle anderen getesteten Materialien im selben Experiment verhielten sich dagegen nicht gleich zum Patientenmaterial.

### Figur 1:

A) Die mittlere Fehlerrate (Mean error rate) wurde für verschiedene cfDNA-ähnliche Kontrollmaterialien (RM) und cfDNA von gesunden Spendern (schwarzer Balken, HD cfDNA) mithilfe des MRD-Tests (supra) ermittelt. Die Proben wurden bei vergleichbaren Zieltiefen von ~100.000× sequenziert.
B) Die im hier beschriebenen Verfahren hergestellte cfDNA (siehe Linie zu BDXXP4 n-cfDNA) weist eine Größenverteilung auf, die der gesunden Patienten cfDNA (schwarze durchgezogene Linie, Healthy donor (HD) cfDNA) am ähnlichsten ist.

Beispiel 2: Validierung eines diagnostischen Verfahrens zur Erkennung einer genetischen Veränderung im Rahmen einer Schwangerschaftsuntersuchung.

Die angeführten Technologien wie PCR und NGS-Verfahren können auch dafür verwendet werden, Erkrankungen eines ungeborenen Kindes im Blut der Mutter nachzuweisen. Dies kann durch eine Flüssigbiopsie erfolgen und anschließender DNA-Extraktion aus dem Blut. Die extrahierte DNA enthält neben der mütterlichen DNA häufig einen Anteil kindlicher DNA. Der Anteil kindlicher DNA liegt häufig im Bereich zwischen 1-10%. Die kindliche DNA unterscheidet sich von der mütterlichen DNA unter anderem durch die Größe. Kindliche DNA ist im Durchschnitt kleiner als mütterliche DNA. Durch die vorliegende Erfindung kann daher Technologie, die extrahierte DNA aus Müttern untersucht, validiert werden. Dies geschieht indem z.B. zwei unterschiedlich in der Länge fragmentierte DNA in einem Verhältnis miteinander vermischt werden, dass dem natürlichen Verhältnis ähnlich ist.

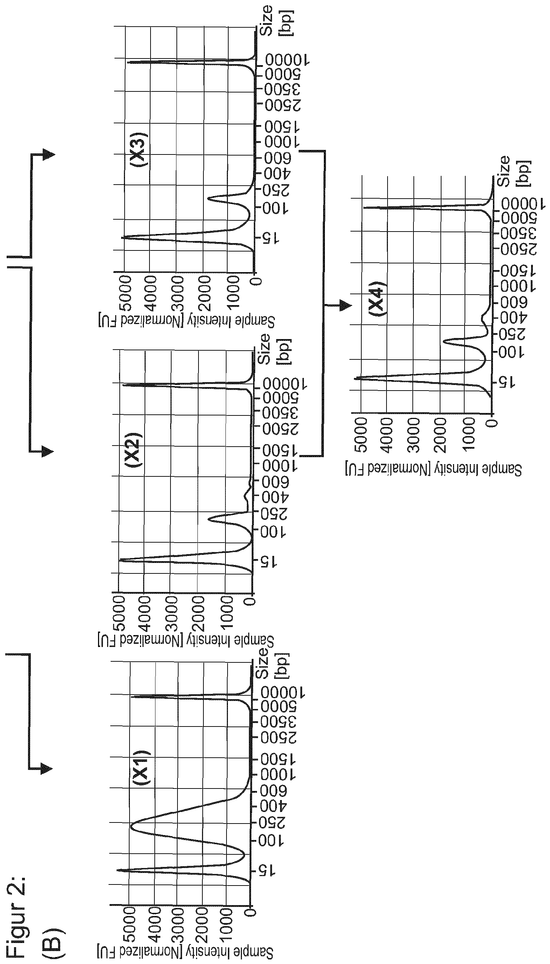

Im vorliegenden Beispiel wurde in einem Experiment untersucht, ob eine Mischung von zwei in der Länge unterschiedlich fragmentierter DNA (siehe Figur 2B X2 und X3) in einem PCR-Verfahren voneinander unterschieden werden können. Es zeigt sich, dass die einzelnen DNA in der Mischung (siehe Figur 2B X4) durch PCR-Verfahren voneinander unterschieden werden können.

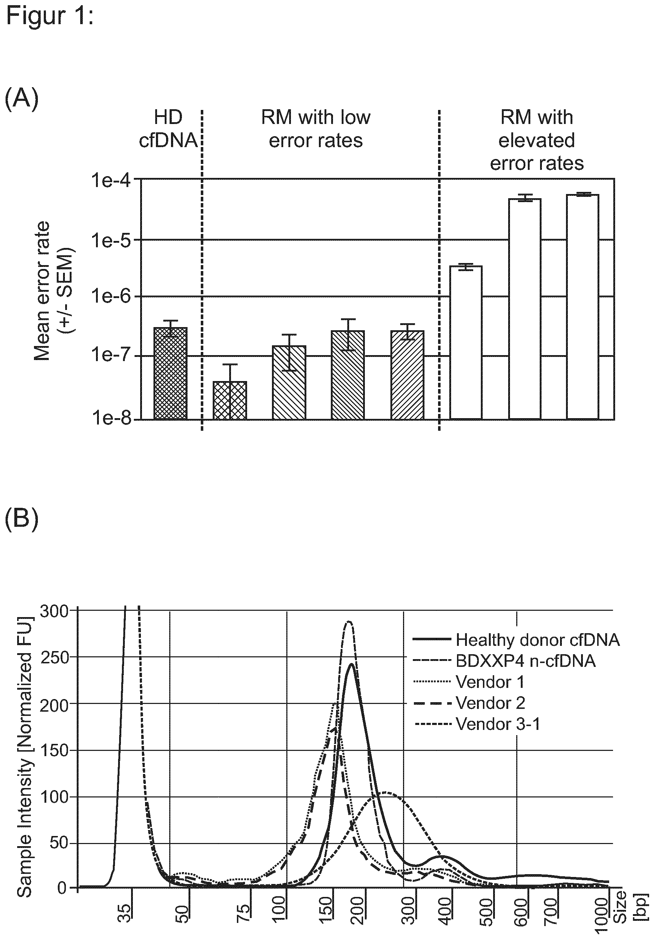

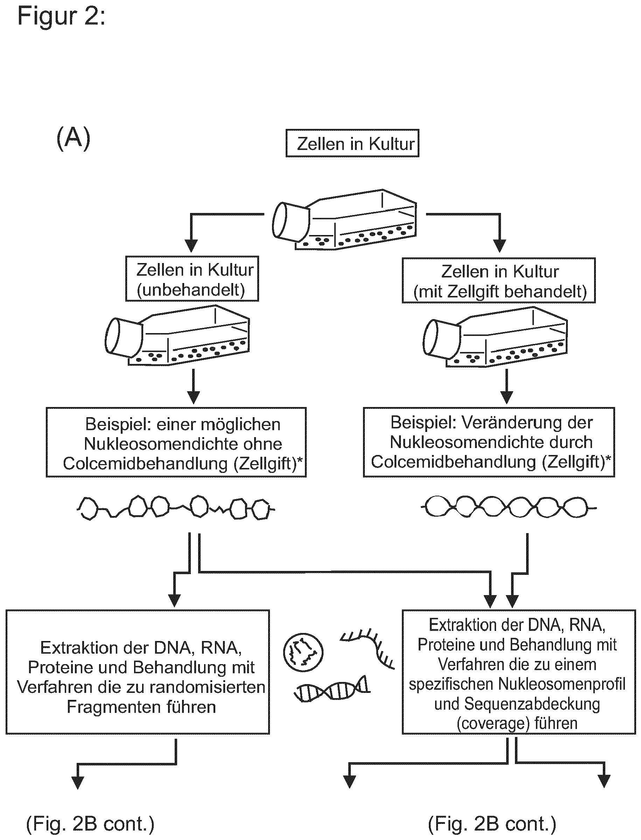

Die Figur 2 zeigt:
(A) Mögliche Unterschiede in der Nukleosomendichte bei Behandlung mit einem oder keinem Zellgift;
(B) Mögliche Fragmentierungsprofile nach Behandlung mit Verfahren die zu randomisierten DNA-Fragmenten führen (X1 z.B. Ultraschall), oder Behandlung der DNA mit Nukleasen, die zu cfDNA Fragmentierungsprofilen, wie bei cfDNA gesunden Menschen (X2) und kranker Menschen (X3) führen. X4 = Überlagerung von X2 und X3.

*Andere Zellgifte können eine andere Nukleosomendichte erzeugen. Dies beeinflusst nach Behandlung mit einer entsprechenden Nuklease das Coverage Profil.

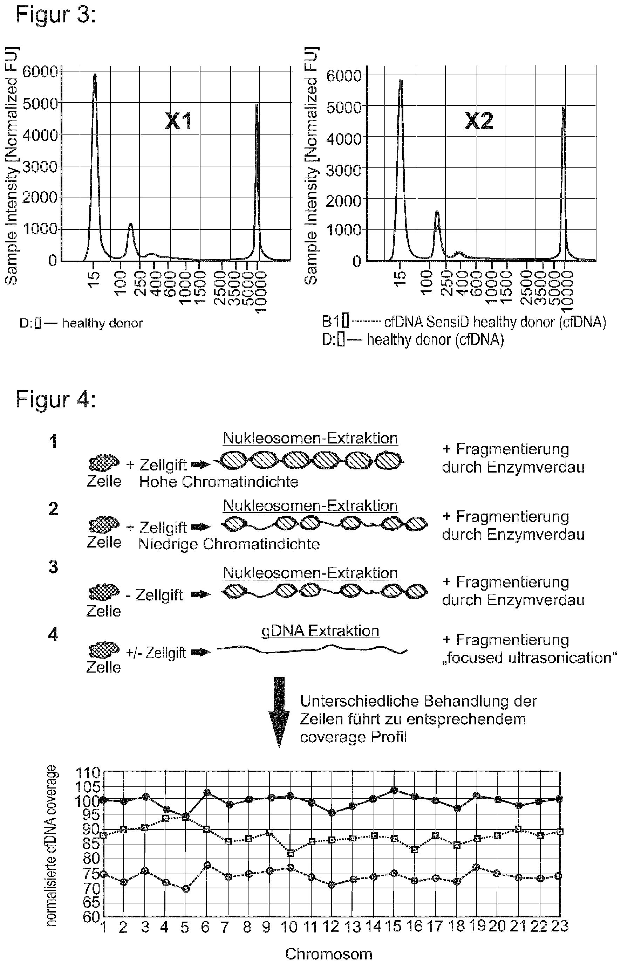

Figur 3 zeigt (X1) die normale Fragmentlängenverteilung im Menschen und (X2) Überlagerung mit dem erfindungsgemäßen Verfahren hergestellter DNA.

Figur 4: Mögliche Herstellverfahren fragmentierter DNA.

DNA kann entweder durch (1,2) Nukleaseverdau von DNA aus Zellen, die mit einem Zellgift behandelt wurden, hergestellt werden, oder (3) durch Nukleaseverdau von DNA aus Zellen, die nicht mit einem Zellgift behandelt wurden, oder durch (4) Verfahren, die nach gDNA-Extraktion zu randomisierten Fragmenten führen. Dabei kann das Verfahren wie in (1,2) dargestellt mit Zellgiften stattfinden, die zu einem Arrest in unterschiedlichen Zellzyklusphasen führen. Wenn Zellgifte, die den Zellzyklus in einer Phase arretieren, die zu einer hohen Chromatindichte führen, eingesetzt werden, wie im Verfahren wie in (1) dargestellt, führt dies zu einer hohen Coverage. Wenn Zellgifte eingesetzt werden, die den Zellzyklus in einer Phase arretieren, die zu einer niedrigen Chromatindichte führen eingesetzt werden, wie im Verfahren wie in (2) dargestellt, führt dies zu einer niedrigen Coverage. (1) und (2) haben den Vorteil, dass zwischen verschiedenen Herstellchargen die gleiche Coverage bezogen auf definierte Sequenzen erreicht wird, während das durch Verfahren wie in (3) beschrieben nicht erreicht wird.

Folglich wird eine bessere Reproduzierbarkeit in der Herstellung durch den erfindungsgemäßen Einsatz von Zellgift und daher eine definierte Coverage erzielt. Der besondere Vorteil von (1) in der Abbildung ist, dass eine höhere und gleichmäßigere Coverage über die gesamte untersuchte Sequenz erreicht wird. Durch den Einsatz von z.B. Colchicin sind die meisten Sequenzen um Histone gewickelt (maximal kondensiert). Dadurch ist die Coverage über das gesamte Genom sehr hoch.

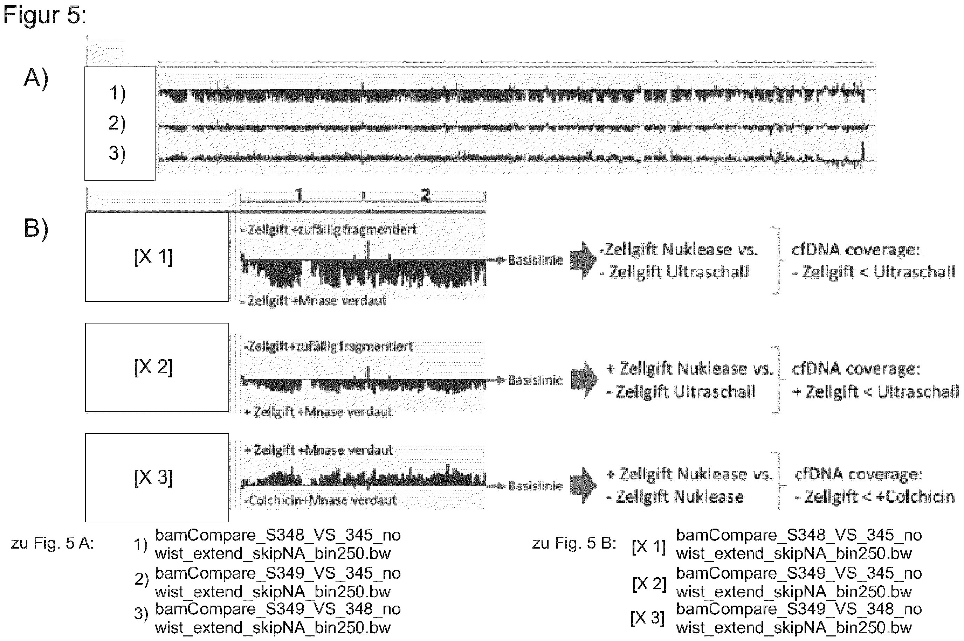

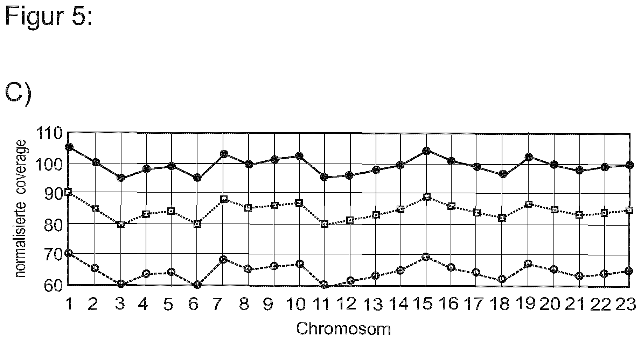

Figur 5 zeigt einen Coverage Vergleich zwischen mit Zellzyklusinhibitor (+Zellgift) behandelten und nicht Zellzyklusinhibitor behandelten (-Zellgift) Zellen nachdem die DNA auf unterschiedlichen Wegen fragmentiert wurde.
(A) Coverage-Verhältnis zwischen den einzelnen Herstellmethoden in der Gesamtgenomübersicht. Es wurden Whole Exome Sequencing (WES) Daten erhoben. Der Vergleich zeigt Unterschiede in der Coverage zwischen DNA aus Zellen, die mit einem Zellgift behandelt wurden, welches zu einer hohen Chromatindichte führt (+ Zellgift) oder ohne Zellgift (- Zellgift) behandelt wurden und anschließend mittels Mnase verdaut wurden. Im Vergleich dazu ist die Coverage bei zufällig fragmentierter DNA am höchsten.
(B) Detailvergleich bei Betrachtung von Chromosom 1 und 2. [X1] vergleicht die Coverage zwischen nicht mit Zellgift (- Zellgift + Nuklease) behandelter Zellen deren gDNA zufällig fragmentiert wurde mit Nuklease verdauter DNA aus Zellen, die nicht mit Zellgift behandelt wurden. Die Coverage ist bei Zellen, die nicht mit Zellgift behandelt wurden, niedriger, hier dargestellt durch die Abweichung von der Basislinie nach unten. [X2] vergleicht die Coverage zwischen nicht mit Zellgift behandelter Zellen deren gDNA zufällig fragmentiert wurde mit Nuklease verdauter DNA aus Zellen die mit Zellgift (+ Zellgift + Nuklease) behandelt wurden. Die Coverage ist bei Zellen, die nicht mit Zellgift behandelt wurden, niedriger, hier dargestellt durch die Abweichung von der Basislinie nach unten. [X3] vergleicht die Coverage zwischen nicht mit Zellgift behandelter Zellen deren DNA mit Nuklease (- Zellgift + Nuklease) verdaut wurde, mit Nuklease verdauter DNA aus Zellen die mit Zellgift (+ Zellgift + Nuklease) behandelt wurden. Die Coverage ist bei Zellen, die mit Zellgift behandelt wurden, höher, hier dargestellt durch die Abweichung von der Basislinie nach oben.
(C) Vereinfachte Darstellung der Coverage zueinander (blau = zufällig fragmentierte DNA, orange = + Zellgift + Mnase Verdau, grau = - Zellgift + Mnase Verdau)

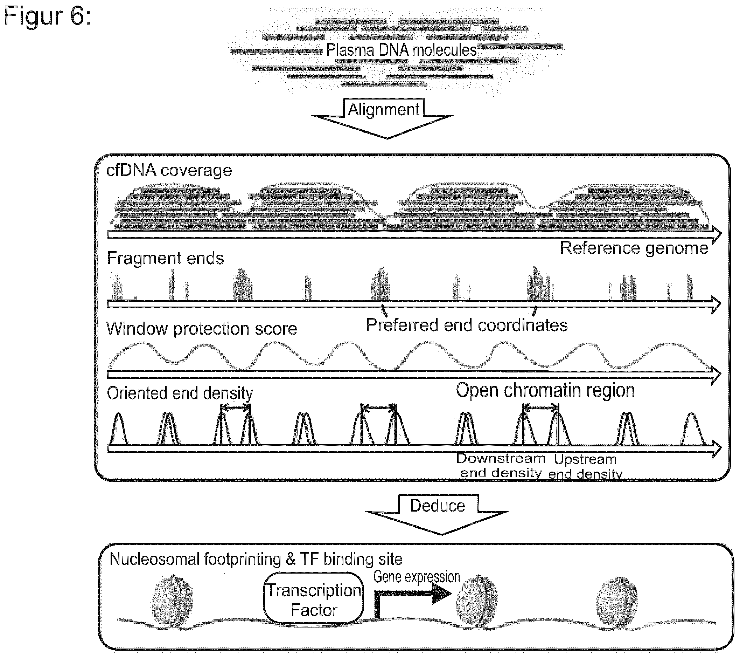

Die Figur 6 aus (8) zeigt den Zusammenhang zwischen der cfDNA Coverage, den präferierten Endstellen (engl. Preferred end coordinates), sowie dem window protection score und den Bereichen des offenen Chromatins (engl. Open Chromatin regions).

Wenn bei Pools fragmentierter DNA (hier plasma DNA molecules) ein Alignment (auch Sequenzalignment; Sequenzalignment bezeichnet den methodischen Vergleich zweier oder mehrerer Nukleotid- oder Aminosäuresequenzen in linearer Abfolge.) durchgeführt wird, häufen sich ihre Enden an spezifischen genomischen Stellen, den so genannten bevorzugten Endstellen, die zwischen DNA-Molekülen, die aus verschiedenen Geweben stammen variabel sein können. Der "Window protection score" (berechnet als die Anzahl der vollständigen Fragmente abzüglich der Anzahl der Fragment Endpunkte innerhalb eines bestimmten "Window"), vermittelt Informationen über den DNA-Schutz gegenüber der Verdauung von Nukleasen, woraus man auf die Nukleosomen-Positionierung schließen kann. Die genomische Abdeckung spiegelt die Chromatinstruktur des Ursprungsgewebes wider.

### Literatur:

1. Snyder MW, Kircher M, Hill AJ, Daza RM, Shendure J.: Cell-free DNA comprises an in vivo nucleosome footprint that informs its tissues-of-origin. Cell 2016;164:57-68
2. Jiang P, Sun K, Tong YK, Cheng SH, Cheng THT, Heung MMS, et al.: Preferred end coordinates and somatic variants as signatures of circulating tumor DNA associated with hepatocellular carcinoma. Proc Natl Acad Sci USA 2018;115:E10925-33.
3. Ivanov M, Baranova A, Butler T, Spellman P, Mileyko V.: . Non-random fragmentation patterns in circulating cell-free DNA reflect epigenetic regulation. BMC Genomics 2015;16:S1
4. Hardham, A.R., Gunning, B.E.S. Some effects of colchicine on microtubules and cell division in roots of Azolla pinnata . Protoplasma 102, 31-51 (1980).
5. Dane, F., Dalgi 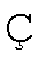 , Ö. The Effects of Fungicide Benomyl (Benlate) on Growth and Mitosis in Onion (Allium cepa L.) Root Apical Meristem. BIOLOGIA FUTURA 56, 119-128 (2005)
6. Sun K, Jiang P, Cheng SH, Cheng THT, Wong J, Wong VWS, Ng SSM, Ma BBY, Leung TY, Chan SL, Mok TSK, Lai PBS, Chan HLY, Sun H, Chan KCA, Chiu RWK, Lo YMD. Orientation-aware plasma cell-free DNA fragmentation analysis in open chromatin regions informs tissue of origin. Genome Res. 2019 Mar;29(3):418-427. doi: 10.1101/gr.242719.118. PMID: 30808726; PMCID: PMC6396422.
7. Sims, David; Sudbery, Ian; Ilott, Nicholas E.; Heger, Andreas; Ponting, Chris P. (2014). "Sequencing depth and Coverage: key considerations in genomic analyses". Nature Reviews Genetics. 15 (2): 121-132. Doi:10.1038/nrg3642. PMID 24434847. S2CID 13325739.
8. Y. M. Dennis Lo, Diana S. C. Han, Peiyong Jiang and Rossa W. K. Chiu (2021). "Epigenetics, fragmentomics, and topology of cell-free DNA in liquid biopsies" Science; Vol 372, Issue 6538; DOI: 10.1126/science.aaw3616

## Patentansprüche

1. Verfahren zur Validierung eines Sequenzierverfahrens für die Bestimmung von cfDNA, **dadurch gekennzeichnet, dass**
i.) ein Referenzmaterial bereitgestellt wird, welches mit den folgenden Schritten hergestellt wird:
a.) Bereitstellen einer Zellkultur aufweisend Zellen,
b.) Behandlung der Zellkultur aus a.) mit einem Hemmstoff zur Zellteilung,
c.) Isolierung der Nukleinsäuren aufweisend Nukleosomen,
d.) Behandlung der Nukleinsäuren aufweisend Nukleosomen mit Nukleasen oder Ultraschall,
e.) Isolierung von Nukleinsäuren mit einer Länge von 20-750 bp, und
ii.) die Nukleinsäuren aus e.) mittels eines Sequenzierverfahrens bestimmt werden.

2. Verfahren zur Validierung eines Sequenzierverfahrens für die Bestimmung von cfDNA nach Anspruch 1, wobei die Zellkultur aufweisend Zellen eukaryotische Zellen, Säugetierzellen oder humane Zellen sind.

3. Verfahren zur Validierung eines Sequenzierverfahrens für die Bestimmung von cfDNA nach einem der vorhergehenden Ansprüche, wobei die Zellkultur aufweisend eukaryontische Zellen ein oder mehrere bestimmte Zelltypen sind ausgewählt aus der Gruppe Körperzellen, Stammzellen, B-Zellen, T-Zellen, natürliche Killerzellen, kranke oder gesunde Zellen, Tumorzellen, polyklonale Zellen, monoklonale Zellen, immortalisierte Zellen.

4. Verfahren zur Validierung eines Sequenzierverfahrens für die Bestimmung von cfDNA nach einem der vorhergehenden Ansprüche, wobei der Hemmstoff zur Zellteilung ausgewählt ist aus der Gruppe Mitose-Hemmstoffe, Checkpoint-Inhibitoren, insbesondere ausgewählt aus der Gruppe Colcemide (CAS-Nummer: 477-30-5), Benomyl (CAS Nummer: 17804-35-2), Nocodazole (CAS Nummer: 31430-18-9), Paclitaxel (CAS-Nummer: 33069-62-4), Vincristine (CAS-Nummer: 57-22-7), Vinblastine (CAS-Nummer: 865-21-4), 8-Chloroadenosine (CAS Nummer: 34408-14-5), WYE 687 dihydrochloride (CAS Nummer: 1702364-87-1), AZD 5438 (CAS Nummer: 602306-29-6), Pladienolide B (CAS Nummer: 445493-23-2), Temsirolimus (CAS Nummer: 162635-04-3), Tosyl-L-Arginine Methyl Ester (TAME) (CAS-Nummer: 1784-03-8), YC 1 (CAS Nummer: 170632-47-0), Indisulam (CAS Nummer: 165668-41-7) .

5. Verfahren zur Validierung eines Sequenzierverfahrens für die Bestimmung von cfDNA nach einem der vorhergehenden Ansprüche, wobei die Nuklease ausgewählt ist aus der Gruppe MNase (EC Nummer: 3.1.31.1), DNase1L3 (EC-Nummer: 3.1.21.1), DNase I (EC-Nummer: 3.1.21.1) .

6. Verfahren zur Validierung eines Sequenzierverfahrens für die Bestimmung von cfDNA nach einem der vorhergehenden Ansprüche, **dadurch gekennzeichnet, dass** eine Validierungs-, Kalibrierungskurve erhalten wird.

7. Verfahren zur Validierung eines Sequenzierverfahrens für die Bestimmung von cfDNA nach einem der vorhergehenden Ansprüche, **dadurch gekennzeichnet, dass** eine Sequenzierung oder Verfahren ausgewählt aus der Gruppe Pyrosequenzierung, Sequenzierung durch Hybridisierung, Ionen-Halbleiter-DNA-Sequenzierungssystem, Nanoporen-Sequenzierung, Next Generation Sequencing (NGS), sequencing-by-synthesis (SBS), Polymerase-Chain-Reaction (PCR), qPCR (real-time quantitative Polymerase Chain Reaction), digital droplet PCR (ddPCR) durchgeführt wird.

8. Verfahren zur Diagnose einer Erkrankung, wobei eine Bestimmung von cfDNA erfolgt, insbesondere zur Beurteilung des Schweregrades und zur therapiebegleitenden Verlaufsbeurteilung und Therapieentscheidung, **dadurch gekennzeichnet, dass** eine Kalibrierung oder Validierung mittels eines Verfahrens nach einem der Ansprüche 1 bis 7 erfolgt.

9. Verfahren zur Erkennung einer minimalen Resterkrankung, wobei eine Bestimmung von cfDNA erfolgt, **dadurch gekennzeichnet, dass** eine Kalibrierung oder Validierung mittels eines Verfahrens nach einem der Ansprüche 1 bis 7 erfolgt.

10. Verwendung eines Kits zur Durchführung eines Verfahrens nach einem der vorhergehenden Ansprüche, insbesondere zur Durchführung einer Liquid-Biopsie.
